Europäisches Patentamt

European Patent Office

Office européen des brevets

(11) Publication number: **0 609 460 A1**

(12)
# EUROPEAN PATENT APPLICATION
## published in accordance with Art. 158(3) EPC

(21) Application number: **93916320.0**

(22) Date of filing: **28.06.93**

(86) International application number:
**PCT/RU93/00139**

(87) International publication number:
**WO 94/00117 (06.01.94 94/02)**

(51) Int. Cl.⁵: **A61K 31/195**, A61K 31/565, A61K 31/44

(30) Priority: **26.06.92 RU 5049685**

(43) Date of publication of application:
**10.08.94 Bulletin 94/32**

(84) Designated Contracting States:
**AT BE CH DE DK ES FR GB IT LI NL SE**

(71) Applicant: **KISLYAKOVA, Nonna Dmitrievna**
**ul. Turmalinovskaya, 79/1-74**
**Rostov-na-Donu, 344038 (RU)**

(72) Inventor: **KISLYAKOVA, Nonna Dmitrievna**
**ul. Turmalinovskaya, 79/1-74**
**Rostov-na-Donu, 344038 (RU)**

(74) Representative: **Lord, Hilton David et al**
**MARKS & CLERK,**
**57-60 Lincoln's Inn Fields**
**London WC2A 3LS (GB)**

(54) **PHARMACEUTICAL AGENT WITH ANTITUMORAL ACTIVITY.**

(57) The invention relates to medicine, in particular in experimental and clinical oncology and may be used for treatment of hormonosensitive tumors in women as well as in neglected cases at the loss of hormonosensitivity. The proposed pharmaceutical agent consists, in the case of treatment of hormonosensitive tumors at women, of a mechanical mixture of 2-4 g of DL-valine and a small nontherapeutic dose of 0.020-0.025 g of methyl-testosterone, and, in the case of treatment of non-hormonosensitive tumors, of a mechanical mixture of 2-4 g of DL-valine, 0.005-0.010 g of methandrostenolone and 0.3-0.5 g of phtivazid. The technical result of using the proposed agent consists in that even in neglected cases the size of tumor decreases, sometimes up to complete resorption, the pain syndrome disappears, the life of incurable patients is substantially prolongated accompanied by a satisfactory subjective condition, or, sometimes, by the resorption of the tumor. The use of the agent is not followed by any toxic effects, it increases even the antitumor resistance of the organism in the form of normalization of hemopoiesis, weight gain, substantial increase of the working capacity. The absence of side-effects and complications allows the agent to be used under ambulant conditions.

EP 0 609 460 A1

The invention pertains to medicine, in particular, to experimental and clinical oncology and may be used for treatment of the hormone-sensitive malignant tumors in women as well as in cases of the neglected oncological process when these tumors lose their sensitivity to hormones.

Pharmaceuticals are known which in experiments in vitro exert cytotoxic effects on animal and human malignant cells present as suspensions of individual cells in the culture medium or as a cell monolayer to tissue culture (see Inventor's certificate for invention No. 750787 of 28.03.1980 A 61 K 31/195 "A pharmaceutical exerting antitumor activity in vitro" by N.D. Kislyakov and co-authors).

These are chemical substances of simple composition and methods of preparation, synthetic DL-amino acids possessing a broad spectrum of cytotoxic effects on certain human and animal malignant cells: DL-alanine, DL-leucine, DL-phenylalanine, DL-glycine, DL-methionine, DL-tryptophan, end DL-valine.

In contrast to others, the synthetic amino acid DL-valine in the experiments in vitro exerts the strongest cytotoxic effect on mammalian cancer cells without any damage of non-malignant cells.

However, the main and very significant shortcoming of the above-mentioned drugs, among them DL-valine, consists in their capacity to damage only individual malignant cells and to exert no such effect in experiments in vivo when these cells form dense cancer tumors in mammals. These drugs are not assimilated by tumor cells as readily as by free floating cells in the culture medium containing them. Therefore the abovesaid drugs require certain conductors which could assist cancer cells of dense tumors in mammals to assimilate them.

Pharmaceuticals are also known which we have selected as the prototype and which exert an antiblastic effect on the hormone-sensitive malignant tumors in women: mammary gland cancer end ovary cancel (See Mashkovsky, M.D. in "Pharmaceuticals", "Medizina" Publishers, M., 1985, part I, p. 598-603 and part II, p. 470-471). These are synthetically obtained steroid hormones, derivatives of a strong androgen, testosterone, the male sex hormone, Among these pharmaceuticals, the following have found the widest clinical application for treatment of hormone-susceptible mammary gland cancer and ovary cancer in women; testosterone propionate, medrotestrone propionate (or 2α-methyldihydrotestosterone),prolotestone (a mixture of 2α-methyldihydrotestosterone ethers). Oil solutions of these drugs are inoculated intramuscularly. In long-term treatment, sometimes another derivative to testosterone, methyltestosterone, is given because it has a weaker antiblastic effect but is advantageous because of its oral (sublingual) administration. All those hormones are usually administered in very large doses: from 50 to 100 mg daily, and are used mainly in highly advanced stages with metastases of mammary gland and ovary cancerns. All the above-mentioned drugs, being testosterone derivatives, exert a more or less marked androgenic effect on the female organism, resulting, after a long-term treatment with large doses, in severe side-effects, the development of general toxicity leading to the following complications: disturbance of water-electrolyte metabolism, severe disorders in the functions of the liver, kidneys, heart, hypercalcemic syndrome, as well as another severe complication for women: signs of virilism associated with a high libido.

For the verification of the antitumor effect these pharmaceuticals were originally tested experimental rats with DMBA-induced mammary gland cancer by subcutaneous or intramuscular injections of oil solutions of the drugs in a dose of 2-3 mg daily per a rat weighing 150-200 g, that is about 15 mg/kg body weight (see Beskrovnt, A.M. "Anti-tumor efficacy of hormones in mammary gland cencer is rats", Voprosy oncologii, 1971, v. XVII, No. 9, p. 95-98). To the same group of antitumor drugs belongs methyltestosterone administered orally the antiblastic effect of which is 2-3 times weaker than that of oil-soluble testosterone derivatives; therefore its experimental therapeutic dose is about 25-40 mg/kg per day. Despite their antitumor effect on the hormone-susceptible induced mammary gland cancer in rats, all these synthetic derivatives of testosterone revealed in the experiments a number of significant shortcomings.

1. In the presence of several cancer tumors of the mammary gland in one animal, mainly small "additional" cancer tumors underwent resolution whereas the "main" tumors which had achieved a controllable size of about 4.0 cm$^3$ were considerably more resistant to the treatment.

2. Regression of the tumors in many cases did not proceed to the end, and inoculation of the drug resulted only in the inhibition of the growth, a remission, followed by new irreversible growth of the same tumors.

3. If a synthetic androgen produced complete regression of the tumor, relapses of tile tumor growth were later observed on the same site.

4. Repeated use of the synthetic androgen which had originally caused regression for treatment of newly growing tumors after remission or relapsing tumors produced no antitumor effect, that is, susceptibility to this drug was lost completely.

5. With three oil-soluble testosterone derivatives; testosterone propionate, 2α-methyldihydrotestosterone propionate, and 2α -methyldihydrotestosterone, more active than methyltestosterone, the per cent of general regression of all tumors was 40%, 70% and 80%, respectively, per cent of complete regression

2

(disappearance of tumor) 10%, 50% and 20%, respectively, and per cent of partial decrease of tumors 30%, 20% and 60%, respectively, for each drug after 30 days of administration. For an effective antitumor action these values are low: there were 60%, 30% and 20% of tumors which did not regress at All for each preparation, respectively.

Taking into account all the above-mentioned shortcomings of the prototype, we propose a new pharmaceutical producing a high clinical effect.

The pharmaceutical exhibiting the antitumor activity consists of a mechanical mixture of 2-4 g of DL-valine and a low, nontherapeutic dose (0.020-0.025 g) methytestosterone for use in cases of hormone-susceptible cancer tumors in women and of a mechanical mixture of 2-4 g of DL-valine, 0.005-0.010g of methandrostenolone, and 0.3-0.5 g of phthivazid for use in cases of cancer tumors not susceptible to hormones.

Thus, the invention is new because it is unknown at the current level of medicine development. The inventive level of the proposed pharmaceutical possessing antitumor activity does not clearly follow from the level of development of current chemotherapy of the hormone-susceptible malignant tumors in women (the prototype). The invention may practically be used on a large scale for successful therapy in outpatient wards of hormone-susceptible malignant tumors in women, even in far advanced cases, when this hormone-susceptibility may be completely lost and all the conventional methods of chemo-hormone-therapy become ineffective.

The successful application of the new pharmaceutical may be due to its strong antiblastic properties, its effective action being completely harmless, as well as its property to enhance the patient's defence forces and to exert a strong analgetic effect.

The essence of the invention is presented by the entirety of the essential features sufficient for the achievement of the necessary result.

For theoretical substantiation of the properties of the alleged pharmaceutical and confirmation of the novelty and the inventive level of the proposed invention, the new antitumor drug was tested experimentally.

When used for treatment of hormone-susceptible DMBA-induced mammary gland cancer tumors in female rats, the proposed pharmaceutical consists of a mechanical mixture of 500-550 mg of synthetic amino acid DL-valine and a low, non-therapeutic dose of 5-20 mg methyltestosterone per kilo body weight of the animal administered daily by the oral route; in cases of long growing mammary gland cancer tumors in rats with lost hormone-susceptibility the new antitumor drug consists of a mechanical mixture of 500-550 mg DL-valine , 20-25 mg of anabolic hormone methandrostenolone, and 40-50 mg phthivazid per 1 kg body weight per day.

In all versions of the new antitumor drug application it is necessary to keep a protein-rich and fat-poor diet.

The experimental data obtained in the studios of the inmost mechanisms of the antiblastic effect on cancer tumors of the new antitumor drug in the form of mechanic mixtures of amino acid DL-valine and low non-therapeutic doses of androgens - methyltestosterone or methandrostenolone (an anabolic with very weak androgenic properties) showed the mediatory role of these hormones for DL-valine required for the assimilation thereof by cancer cells forming dense tumors in mammals.

The limit quantitative values of the mixture components depend on the degree of activity of the entire antitumor preparation. As shown by the experimental studies, the optimal dose of DL-valine is 550 mg/kg, its decrease to 500 mg/kg has no influence on its effect, neither does a further increase of the dose, for instance by 2-fold. A decrease of DL-valine dose below 500 mg/kg worsens the antiblastic effect of the mixture and with a dose 2-fold lower than the optimal (275 mg/kg) the regression effect disappears completely. The non-therapeutic dose of methyltestosterone of 5 mg per kilo body weight in combination with DL-valine gives the optimal antiblastic effect on induced cancer tumors of the size of 4.0 $cm^3$ to 6.0 $cm^3$ which still have very high hormone-susceptibility. With further growth of the tumors the hormone-susceptibility declines, and for tumor sizes over 6.0 $cm^3$ up to 8-10 $cm^3$ it was found better to use DL-valine in combination with methyltestosterone, 10 mg/kilo body weight.

These optimal doses of methyltestosterone are considered to be nontherapeutic because they alone, without DL-valine, cause no regression or even inhibition of the growth of the hormone-susceptible mammary gland cancer tumors in rats. Methyltestosterone alone causes regression of such experimental tumors only beginning with a dose of 25 mg/kg body weight, and regression is this case shows all the above-mentioned shortcomings of the antiblastic effect of oil-soluble testosterone derivatives (the prototype). According to our data, the mechanical mixture of DL-valine with 25 mg/kg methyltestosterone has the same shortcomings of the antiblastic effect but less marked than in the prototype. A dose of methyltestosterone below 5 mg/kg in combination with DL-valine produce a significantly lower regression of the hormone-susceptible cancer tumors in rats.

In experiments with similar long-term growing tumors 10 cm$^3$ or larger in size which have partially or completely lost their androgen susceptibility a mechanical mixture of DL-valine with another very weak androgen, methandrostenolone, in a dose of 20-25 mg/kg body weight was found to be optimal. A 2-fold decrease of the dose (12.5 mg/kg) in mixture with DL-valine instead of regression caused insignificant inhibition of growth of these cancer tumors and an increase had no effect on the results.

However, regression of experimental cancer tumors in rats treated with a mixture of DL-valine with methandrostenolone alone was extremely slow, so that in, 30 days of the experiment all the tumors regressed only by 30%-71% of its original size, and on the average by only 52% (Table 1).

Phthivazid, a known drug for tuberculosis with a strong membranotropic effect, was found to be capable to increase considerably the conductivity of DL-valine by methandrostenolone into the cells of long-term growing dense tumors of rat mammary glands. The optimal dose of phthivazid was found empirically to be 45-50 mg/kg body weight. A considerable increase or decrease of the dose either does not affect the tumor growth or may even enhance it.

Table 1

A phramaceutical exerting antotumoral activity
White random-bred rats with induced mammary gland cancer.

Treatment: DL-valine (550 mg/kg) and methandrostenolone (25 mg/kg) in comparison with untreated tumor-bearing animals (conditional tumor sizes in $cm^3$)

| Rat No. | Initial data | Intervals | | | |
|---|---|---|---|---|---|
| | | 1 week | 2 weeks | 3 weeks | 4 weeks |
| CONTROL GROUP (untreated) | | | | | |
| 1 | 4 | 7.8 | 20.2 | 37.3 | died |
| 2 | 3.9 | 5.7 | 7.0 | 13.3 | 20.2 |
| 3 a) | 6.0 | 10.2 | 16.0 | 25.1 | died |
| b) | 5.0 | 5.0 | 5.3 | 8.2 | |
| 4 | 5.1 | 6.6 | 7.1 | 7.8 | 8.0 |
| 5 | 7.3 | 12.5 | 19.0 | 30.2 | 42.0 |
| 6 | 8.0 | 9.6 | 20.0 | 36.0 | died |
| 7 | 3.8 | 4.5 | 9.5 | 14.2 | 23.0 |
| 8 | 4.5 | 6.2 | 7.1 | 8.3 | 10.3 |
| Arithmetic mean and variation ranges | 5.3 | 8.7 | 11.6 | 20.2 | 20.7 |
| | 3.8-8.0 | 4.5-12.5 | 5.3-20 | 7.8-37.3 | 8.0-42.0 |
| P: comparison with initial data | | <0.05 | <0.01 | <0.01 | <0.01 |

PROGRESSION

## Table 1 (continued)

| Rat. No. | Initial data | Intervals | | | | % regression |
|---|---|---|---|---|---|---|
| | | 1 week | 2 weeks | 3 weeks | 4 weeks | |

**EXPERIMENTAL GROUP (DL-valine and methandrostenolone)**

| Rat. No. | Initial data | 1 week | 2 weeks | 3 weeks | 4 weeks | % regression |
|---|---|---|---|---|---|---|
| 1 | 4.6 | 4.4 | 3.4 | 2.1 | 2.0 | 56 |
| 2 | 10.5 | 11.2 | 6.9(necrosis) | 6.1 | 5.0 | 52 |
| 3 | 5.5 | 5.7 | 4.7 | 2.8 | 2.3 | 60 |
| 4 | 7.2 | 3.4 | 2.6 | 2.7 | 2.1 | 71 |
| 5 | 5.2 | 3.0 | 2.8 | 2.5 | 2.2 | 57 |
| 6 | 7.7 | 5.8 | 5.5 | 5.8 | 5.5 | 30 |
| 7 | 6.6 | 6.3 | 5.6 | 4.6 | 4.2 | 36 |
| 8 | 6.3 | 5.0 | 4.0 | 4.3 | 3.3 | 50 |
| 9 | 4.5 | 3.7 | 3.6 | 2.8 | 2.0 | 53 |
| Arithmetic mean | 6.5 | 5.3 | 4.3 | 3.7 | 3.1 | 52 |
| and variation ranges | 4.5–10.6 | 3.0–11.2 | 2.6–6.9 | 2.1–6.1 | 2.0–5.5 | 30–71 |
| P | – | $> 0.05$ | $< 0.05$ | $< 0.01$ | $< 0.01$ | |

REGRESSION

P – comparison of each week with the initial data.

A comparative analysis with the prototype - oil-soluble synthetic testosterone derivatives administered parenterally - done, on the basis of the experimental data showed the new alleged antitumor drug to be significantly different from the known ones.

1. The alleged antitumor drug exerts the antiblastic effect by the close interaction of 2 components of a mechanical mixture: DL-valine amino acid capable of producing an idependent cytolysing effect only on individual mammaliam cancer cells in vitro and an androgen capable of performing in a small non-therapeutic dose the role of the conductor of this amino acid into the cells of a solid mammalian malignant tumor.

2. The alleged drug in the mechanical mixture with DL-valine amino acid contains, in comparison with the prototype(25-40 mg/kg), a low (5-10 mg/kg) nontherapeutic dose of a testosterone derivative, methyltestosterone, exerting no antiblastic effect without DL-valine.

3. The new antitumor drug differs considerably from the prototype also by a highly effective antiblastic action:

a) in the presence of several tumors in an animal, the "main" tumors which have achieved a controllable size (4.0 cm$^3$) undergo resolution first or simultaneously with "additional" ones;

b) no discontinuation of regression, or occurrence of remission followed by irreversible tumor growth is observed upon administration of the new preparation;

c) no relapses of tumor growth is observed after complete resolution thereof;

d) in repeated use of the new preparation, no loss of sensitivity to it is observed;

e) daily administration for 30 days of the proposed preparation produces the regression effect related with the degree of tumor hormone-susceptibility and methyltestosterone dose: with a dose of 5 mg/kg per cent of general regression was 89% (10% of tumors did not resolve - 2 out of 17); complete regression (disappearance of tumors) was observed with 65% of tumors (11 out of 17) and partial regression (by 53%-85% of the initial size of the tumor) with 24%, that is 4 tumors out of 17 (see Table 2); with a dose of 10 mg/kg general regression was observed with 100% of tumors, that is, all the tumors resolved on the average by 80%, complete regression with 20%, and, in 80% of the tumors resolution achieved 61% to 95% of the initial size of the tumor within 30 days of the experiment (see Table 3).

4. The alleged antitumor preparation, in contrast to the prototype and other known cytostatics, alongside with the resolving effect on malignant tumor is also capable to enhance the defence forces, e.i. resistance to tumor of the tumor bearer, as indicated by:

a) a significant increase in the body weight of the animal accompanying the regression (see Table 4);

b) haemopoiesis stimulation leading to normalization thereof in the course of tumor regression (see Table 5);

c) a significant increase (2-3-fold) of the survival time of the animals in cases of insignificant antitumor effect achieved in 30 days of the experiment as compared with that of control (untreated) animals after termination of the experiment.

Table 2

A pharmaceutical exerting antitumoral activity Induced mammary gland cancer in random-bred white female rats. Conditional tumor sizes (in cm³) in treatment with 5 mg/kg methyltestosterone and 550 mg/kg DL-valine

| Rat. No. | Initial data | Tumor sizes after intervals of | | | |
|---|---|---|---|---|---|
| | | 1 week | 2 weeks | 3 weeks | 4 weeks |
| CONTROL GROUP (untreated) | | | | | |
| 1 | 6.4 | 7.5 | 11.5 | 18.4 | 21.3 |
| 2 | 4.6 | 6.4 | 7.1 | 7.1 | 7.3 |
| 3 | 7.2 | 8.4 | 8.4 | 14.3 | 17.2 |
| 4 | 7.2 | 12.0 | 19.2 | 30.5 | 59.4 |
| 5 | 4.1 | 9.3 | 22.1 | 36.4 | died |
| 6 | 3.9 | 6.22 | 23.4 | died | - |
| 7 | 3.8 | 5.3 | 9.5 | 15.8 | 21 |
| 8 | 3.9 | 5.8 | 5.9 | 6.7 | 9.6 |
| | 0 | 1.8 | 2.7 | 4.7 | 9.7 |
| 9 | 6.9 | 12.5 | 13.3 | 18.1 | 21 |
| 10 | 4.3 | 6.3 | 20.5 | 35.5 | died |
| No. of rats/Tumors | 10/11 | | | | |

Table 2 (continued)

| | Rat. No. | Initial data | Tumor sizes after intervals of | | | |
|---|---|---|---|---|---|---|
| | | | 1 week | 2 weeks | 3 weeks | 4 weeks |
| Arithmetic mean and variation ranges 1. | | 5.2 (3.8-7.2) | 7.5 (1.8-12.5) | 12.7 (2.7-23.4) | 18.7 (4.69--36.4) | 20.8 (7.3--59.4) |
| P in comparison with initial data (be weeks in control group) 2. | | | $<0.05$ | $<0.01$ | $<0.01$ | $<0.01$ |
| P in comparison of control and experimental animals (by weeks) 3. | | | $>0.05$ | $<0.01$ | $<0.01$ | $<0.01$ | $<0.01$ |

EXPERIMENTAL GROUP (treated with the preparation)

| Rat. No. | Initial data | Tumor sizes after intervals of | | | | % regression |
|---|---|---|---|---|---|---|
| | | 1 wks | 2 wks | 3 wks | 4 wks | |
| 1 | 4.4 | 2.0 | 0.81 | 0 | 0 | 100 |
| 2 | 4.2 | 2.2 | 0.7 | 0.4 | 0 | 100 |
| 3 | 4.4 | 1.0 | 0.04 | 0 | 0 | 100 |
| 4 | 4.0 | 0.8 | 0.04 | 0 | 0 | 100 |
| 5 a) | 5.6 | 2.8 | 0 | 0 | 0 | 100 |
| b) | 1.0 | 0.8 | 0 | 0 | 0 | 100 |
| 6 | 4.9 | 4.7 | 3.8 | 2.8 | 1.0 | 80 |
| 7 | 4.8 | 0.4 | 0.1 | 0 | 0 | 100 |
| 8 a) | 6.4 | 5.3 | 4.8 | 2.9 | 3.0 | 53 |
| b) | 0.2 | 0 | 0 | 0 | 0 | 100 |
| 9 | 4.7 | 3.5 | 1.1 | 0.1 | 0 | 100 |

Table 2 (continued)

| Rat. No. | Initial data | Tumor sizes after intervals of | | | | % regression |
|---|---|---|---|---|---|---|
| | | 1 wks | 2 wks | 3 wks | 4 wks | |
| 10 | 5.7 | 3.2 | 2.2 | 1.0 | 0 | 100 |
| 11 | 7.6 | 6.5 | 3.9 | 3.5 | 2.0 | 74 |
| 12 | 4.7 | 3.2 | 2.3 | 1.28 | 0.75 | 85 |
| 13 | 7.8 | 4.6 | 5.1 | 2.0 | 0 | 100 |
| 14 a) | 6.2 | 7.3 | 7.5 | 8.7 | 10.7 | – |
| b) | 3.1 | 2.7 | 3.6 | 2,5 | 3.5 | – |

No. of rats/tumors $\frac{14}{17}$

| Arithmetic mean and variation ranges 1. | 4.7 (0.2-7.8) | 3 (0-7.3) | 2.1 (0-7.5) | 1.5 (0-8.7) | 1.2 (0-10.5) | 82% (0-100) 11 tumors 100% |

| P in comparison with initial data (by weeks in experimental group) 2. | | <0.01 | <0.01 | <0.01 | <0.01 | 4 tum. >50% 2 tum. 0% (without regression) |

Note: a) several tumors in one rat.
b)

9

### Table 3
A pharmaceutical exerting antitumoral activity
Induced mammary gland cancer in random-bred white mice
(conditional tumor sizes in $cm^3$) treated with 10 mg/kg
methyltestosterone and 550 mg/kg DL-valine

| Rat. No. | Initial data | Tumor sizes after intervals of | | | |
|---|---|---|---|---|---|
| | | 1 week | 2 weeks | 3 weeks | 4 weeks |
| **CONTROL GROUP (untreated)** | | | | | |
| 1 | 4 | 7.8 | 20.2 | 37.3 | died |
| 2 | 3.9 | 5.7 | 7.0 | 13.3 | 20.2 |
| 3 a) | 6.0 | 10.2 | 16.0 | 25.1 | died |
| b) | 5.0 | 5.0 | 5.3 | 8.2 | |
| 4 | 5.1 | 6.6 | 7.1 | 7.8 | 8.1 |
| 5 | 7.3 | 12.5 | 19.0 | 30.2 | 42.0 |
| 6 | 8.0 | 9.6 | 20.0 | 36.0 | died |
| 7 | 3.8 | 4.5 | 9.5 | 14.2 | 23.0 |
| 8 | 4.5 | 6.2 | 7.1 | 8.3 | 10.3 |
| Arithm. mean and variation | 5.3 | 8.7 | 11.6 | 20.2 | 20.7 |
| ranges | (3.8-8.0) | (4.5-12.5) | (5.3-20) | (7.8-37.3) | (8.0-42.0) |
| P as compared with initial data | | $<0.05$ | $<0.01$ | $<0.01$ | $<0.01$ |

### P R O G R E S S I O N

| Rat. No. | Initial data | Tumor sizes after intervals of | | | | % regression |
|---|---|---|---|---|---|---|
| | | 1 week | 2 weeks | 3 weeks | 4 weeks | |
| **EXPERIMENTAL GROUP (DL-valine and methyl-testosterone)** | | | | | | |
| 1 | 6.8 | 3.2 | 0.7 | 0.8 | 0.8 | 88 |
| 2 | 5.0 | 4.3 | 4.0 | 3.1 | 1.8 | 64 |
| 3 | 4.2 | 3.0 | 2.3 | 0.8 | 0 | 100 |
| 4 | 7.0 | 8.6 | 6.9 | 3.9 | 2.0 | 71 |
| 5 | 7.3 | 7.4 | 5.0 | 2.2 | 2.5 | 66 |

Table 3 (continued)

| | | | | | | |
|---|---|---|---|---|---|---|
| 6 | 6.7 | 4.1 | 2.4 | 0.9 | 0.6 | 95 |
| 7 | 7.1 | 5.0 | 2.2 | 1.0 | 0.9 | 87 |
| 8 | 8.2 | 6.9 | 5.0 | 3.3 | 2.8 | 66 |
| 9 | 5.8 | 4.0 | 2.3 | 1.6 | 0 | 100 |
| 10 | 7.5 | 7.8 | 5.6 | 4.1 | 2.9 | 61 |
| Arithm. mean and variation | 6.3 | 5.4 | 3.6 | 2.1 | 1.4 | 80 |
| ranges | (4.2-8.2) | (3.0-8.6) | (0.7-6.9) | (0.8-4.1) | (0-2.9) | (61-100) |
| P as compared with initial data | | >0.05 | <0.01 | <0.01 | <0.01 | 2 tum. 100% all others <50% |

R E G R E S S I O N

Table 4

A pharmaceutical exerting antitumoral activity

Induced mammary gland cancer in random-bred female rats. Weight changes (in g) of experimental rats treated with 5 mg methyltestosterone and 550 mg DL-valine per kg body weight and control animals (untreated) during one month

EXPERIMENTAL GROUP (All animals with tumor regression)

| Rat. No. | Initial | weight gain at intervals of | | | | weight gain vor 1 month |
|---|---|---|---|---|---|---|
| | | 1 week | 2 weeks | 3 weeks | 4 weeks | |
| 1 | 220 | 230 | 240 | 245 | 250 | +30 |
| 2 | 260 | 265 | 270 | 270 | 275 | +15 |
| 3 | 240 | 225 | 260 | 260 | 265 | +25 |
| 4 | 200 | 210 | 215 | 220 | 220 | +20 |
| 5 | 200 | 210 | 220 | 235 | 240 | +40 |

11

Table 4 (continued)

| | | | | | | |
|---|---|---|---|---|---|---|
| 6 | 160 | 130 | 18ⁱ | 190 | 195 | +15 |
| 7 | 200 | 210 | 215 | 220 | 230 | +30 |
| 8 | 240 | 240 | 245 | 250 | 260 | +20 |
| 9 | 220 | 220 | 230 | 235 | 240 | +20 |
| 10 | 240 | 255 | 260 | 260 | 255 | +15 |
| 11 | 170 | 180 | 190 | 190 | 200 | +30 |

| | | | | | | |
|---|---|---|---|---|---|---|
| Arithmet. mean and variation ranges | 213 (160-260) | 220 (180-265) | 230 (185-270) | 234 (190-270) | 239 (195-275) | +23 (15-40) |

P as compared with initial (by weeks)   $>0.05$  $>0.05$  $>0.05$  $<0.05$

CONTROL GROUP (with progression)

| Rat. No. | Ini- tial | in 1 week | in 2 weeks | in 3 wks | in 4 weeks weights of | | | Weight loss in 1 month | |
|---|---|---|---|---|---|---|---|---|---|
| | | | | | ani- mal | tu- mor | ani- mal with- out tumor | ani- mal with- out tu- mor | with tu- mor |
| 1 | 250 | 220 | 240 | 240 | 250 | 20 | 230 | -20 | -0 |
| 2 | 250 | 230 | 230 | 240 | 240 | 6 | 234 | -16 | -10 |
| 3 | 220 | 215 | 290 | 285 | 200 | 20 | 180 | -40 | -20 |
| 4 | 240 | 230 | 220 | 235 | 230 | 50 | 180 | -60 | -10 |
| 5 | 200 | 190 | 195 | 190 | 180 | 20 | 160 | -40 | -20 |
| 6 | 260 | 260 | 250 | 230 | 240 | 20 | 220 | -40 | -20 |

| Arithm. mean and variat- ion ranges | 236 (200-260) | 224 (190-260) | 237 (195-290) | 236 (190-285) | | | 200 (160-234) | -36 (16-60) | -13 (0-20) |
|---|---|---|---|---|---|---|---|---|---|

P as compared with initial (by weeks)   $>0.05$  $>0.05$ $>0.05$   $<0.05$

Table 5

A pharmaceutical exerting antitumoral activity

Stimulation of hemopoiesis accompanying tumor regression in female rats with induced mammary gland cancer treated with DL-valine and methyltestosterone (the non-parametrical method of statistical treatment by the "I" criterion of Wilcockson-Mann-Witney. The mean values and variation ranges are presented).

| Animal groups | Hemoglobin by Sally's test | Erythrocytes in 1 $mm^3$ | Leucocytes in 1 $mm^3$ |
|---|---|---|---|
| 1 | 2 | 3 | 4 |
| I Normal (10 animals) | 87 (84-90) | 4534000 (4020000-4920000) | 5300 (4000-6600) |
| II Controls with tumors (untreated) | 76 (48-96) | 4165000 (3100000-4800000) | 9650 (7500-12000) |
| P in comparison of groups I and II | $> 0.05$ ~ | $> 0.05$ ~ | $< 0.01$ + |
| III Experimental with tumors, treated | 82 (66-94) | 4291428 (3820000-4710000) | 9057 (6900-13500) |
| P in comparison of groups II and III | $> 0.05$ ~ | $> 0.05$ ~ | $> 0.05$ ~ |
| P in comparison of groups I and III | $> 0.05$ ~ | $> 0.05$ ~ | $< 0.01$ + |

Notes: + = increase, − = decrease, ~ = unchanged.

Table 5 (continued)

| Animal groups | Eosino- phils | Stab forms | Segmen- ted cells | Mono- cytes | Lympho- cytes |
|---|---|---|---|---|---|
| 1 | 5 | 6 | 7 | 8 | 9 |
| I Normal (10 animals) | 2.9 (0-6) | 3.4 (2-7.5) | 38.7 (19-56) | 5.6 (2-8) | 52.2 (36-68) |
| II Controls with tumors (untreated) | 2.6 (0-5.0) | 7.2 (4-11) | 62 (56-68) | 1.7 (1-4) | 27.8 (22-35) |
| P in compari- son of groups I and II | $\sim$ >0.05 | + <0.01 | + <0.01 | - <0.01 | - <0.01 |
| III Experimental with tumors, treated | 2.0 (0.5-4.0) | 3.6 (2.0-5.0) | 45 (31-61) | 4.7 (2-8) | 43 (31-59) |
| P in compari- son of groups II and III | $\sim$ >0.05 | - <0.05 | - <0.05 | + <0.05 | + <0.01 |
| P in compari- son of groups I and III | $\sim$ >0.05 | $\sim$ >0.05 | $\sim$ >0.05 | $\sim$ >0.05 | $\sim$ >0.05 |

Notes: + = increase, − = decrease, $\sim$ = unchanged.

## Table 6

### A pharmaceutical exerting antitumoral activity

The effect of a mixture of DL-valine (550 mg/kg), methandrostenolone (25 mg/kg) and phthivazid (50 mg/kg) on large size tumors (or on several tumors in one animal) of induced mammary gland cancer in random-bred white rats .(Conditional tumor sizes in $cm^3$)

| Rat | No. | Initial size | At intervals of (weeks) | | | | | | | % regression |
|---|---|---|---|---|---|---|---|---|---|---|
| | | | 1 | 2 | 3 | 4 | 5 | 6 | | |
| 1 | a) | 18.5 | 8.4 | 10.3 | 11.9 | 11.3 | 6.2 | 8.9 | | 52 |
| | b) | 1.6 | 3.8 | 2.1 | 5.5 | 4.7 | 10.2 | 8.5 | | – |
| 2 | | 24.0 | 29.6 | 23.9 necrosis | 16.5 | 4.4 | 4.9 | 6.5 | | 73 |
| 3 | | | 11.4 | 10.9 | 11.4 | 14.1 | 15.5 | 22.7 | 31.7 | – |
| 4 | a) | 10.8 | 7.6 | 4.3 | both fused | 2 together | 2 together | 2 together | | 75 |
| | b) | 3.2 | 2.7 | 1.0 | -3.4 | -2.5 | -3.0 | -3.5 | | |
| 5 | a) | 12.5 | 6.2 | 4.3 | 1.9 | 1.2 | 0 | 0 | | 100 |
| | b) | 0 | 0.8 | 5.0 | 2.7 | 2.5 | 0 | 0 | | 100 |
| 6 | | 12.0 | 12.2 | 4.4 necrosis | 0.8 | 0 | 0 | 0 | | 100 |
| 7 | a) | 16.5 | 7.3 | 4.3 | 3 tumors fused into one | 3 together | 3 together | | | |
| | b) | 3.7 | 2.9 | 1.6 | | -13.4 | -20.0 | -21.5 | | – |
| | c) | 1.0 | 1.9 | 3.7 | -19.2 | | | | | |
| 8 | a) | 10.1 | 8.9 | 0 necrosis | 0 | 0 | 0 | 0 | | 100 |
| | b) | 1.2 | 1.0 | 1.5 | 1.0 | 1.2 | 1.9 | 1.5 | | – |
| 9 | a) | 11.0 | 2.5 | necr. | 0 | 0 | 0 | 0 | | 100 |
| | b) | 4.2 | 3.4 | 3.0 | 1.5 | 0.9 | 1.0 | 0.6 | | 86 |
| 10 | | | 9.4 | 11.5 | 12.3 | 7.1 | 5.0 | 4.7 | 3.6 | 61 |
| No. of tumors | | 16 | 17 | 17 | 17 | 17 | 17 | 17 | | 14 |

Table 6 (continued)

| Rat No. | Ini-tial size | At intervals of (weeks) | | | | | | % reg-ression |
|---|---|---|---|---|---|---|---|---|
| | | 1 | 2 | 3 | 4 | 5 | 6 | |
| M (variation ranges) and arythmet.mean | 9.4 (1.0-24.0) | 7.0 (0.8-29.6) | 5.5 (0-23.9) | 5.3 (0-19.2) | 3.7 (0-15.5) | 4.4 (0-22.7) | 5.0 (0-31.7) | 1) 4 tum. without regress. 2) 1 tum. with regr. 3) 5 tums with regr. |
| P (comparison with initial data) | | >0.05 | >0.05 | <0.05 | <0.01 | <0.01 | <0.01 | 100% 60.5% (0-100- |

| II. Untreated animals (control) | | | | | | | |
|---|---|---|---|---|---|---|---|
| 1 | 11.5 | 18.4 | 21.8 | 30.5 | 42.5 | died | - |
| 2 | 9.3 | 22.1 | 33.4 | died | - | - | - |
| 3 | 12.5 | 13.3 | 18.1 | 21.0 | 28.7 | 38.3 | died |
| 4 | 12.0 | 19.2 | 30.5 | 42.8 | 50.8 | 59.8 | died |
| 5 | 9.5 | 15.8 | 21.7 | 39.2 | 43.6 | died | - |
| 6 a) | 3.4 | 5.4 | 8.2 | 9.6 | 15.1 | | |
| b) | 5.6 | 7.0 | 8.9 | 10.0 | 12.2 | died | |

| No. of tumors | 7 | 7 | 7 | 6 | 6 | 2 | - |
|---|---|---|---|---|---|---|---|
| M (variation range) | 9.1 (3.4-12.5) | 14.5 (5.7-22.1) | 20.7 (3.2-36.4) | 25.5 (9.6-42.6) | 32.1 (12.2-50.8) | | |
| P (comparison with initial data) | | <0.05 | <0.05 | <0.05 | <0.01 | <0.01 | - |

Notes: 1. When 2-3 tumors fused into one, the statistical treatment took into account 2 or 3 tumors.
2. The tumor size equal to 0 (regression) was considered as one tumor.

5. In contrast to other cytostatic drugs, the alleged pharmaceutical during the process of tumor regression can exert an analgetic effect as was followed in the experiments an a significant increase in the threshold of the pain excitability and, consequently, a decrease in the pain sensitivity in animals. In the absence of regression the analgetic effect ceased, and this may be used clinically for the evaluation of the effect of therapy.

6. In contrast to the prototype, the alleged pharmaceutical may exert the antiblastic effect on the long-term growing cancer tumors partially or completely losing their susceptibility to androgens which may be an experimental model of clinically neglected cancer process. In this case the antitumoral preparation contains, in mixture with DL-valine, an anabolic drug methandrostenolone and a known antituberculosis drug phthivazid which have previously never been used as antitomural drugs. In this composition, the new antitumoral preparation exerting the antiblastic effect on large fused (2-3) and tremendous single experimentally induced tumors (up to 24.0 cm$^3$) causes the regression of the tumors accompanied by frequent necroses. In this case the liquid structureless tumor masses pore our, and healing of normal tissues begins at this site (see Table 6).

7. Morphological lesions which occur in the tumor tissue and result in regression also have their peculiar features: under the influence of the damaging therapeutic effect of any chemical drug and even irradiation there occurs marked fiber formation, whereas the alleged pharmaceutical induces changes due mostly to the lysis of the cells of parenchyma and stroma of the tumor tissue, necrolysis with subsequent resorption (see Fig. 1).

On the basis of the aforementioned experimental data and, especially, taking into account complete harmlessness of the new antitumoral preparation tested and confirmed in normal animals within the framework of the completed pharmacobiological pro-clinical trials we propose for clinical application a new antitumoral pharmaceutical consisting of a mechanical mixture of 2-4 g of DL-valine amino acid and a low non-therapeutical dose 0.020-0.025 g methyltestosterone per day for treatment of hormone-susceptible cancer tumors in women, and a pharmaceutical consisting of a mechanical mixture of 2-4 g of DL-valine, 0.005-0.010 g methandrostenolone and 0.3-0.5 g phthivazid daily for treatment of cancer tumors which have partially or completely lost their hormone-susceptibility.

The daily doses of the proposed pharmaceutical for clinical use have been calculated from the doses used experimentally.

Thus, the dose for DL-valine was calculated to be 2-4 g taking into account the experimental dose of 500-550 mg/kg body weight of animals add at least 10 times lower intensity of amino acid metabolism in man; consequently, about 50-55 mg/kg body weight of man of DL-valine should be used or 2-4 g daily for a person weighing 50-70 kg.

The doses of hormones were calculated in a different way, since human and animal sensitivity to them frequently does not correlate with differences in the metabolism intensity.

A dose of methyltestosterone 20-25 mg or 0.020-0.025 g (4-5 tablets) per day was calculated on the basis of experimental and clinical data according to which its experimental nontherapeutic dose (5 mg/kg body weight) is 5 times lower than the therapeutic one - 25 mg/kg animal body weight. If one takes into account that androgen therapy in clinical cases of mammary gland cancer stipulates a dose of methyltestosterone of 100 mg and more daily (see Bruskin, Ya.M., in "Hormone therapy of mammary gland cancer", "Medizina" Publishers, M., 1969, p. 69-73), the non-therapeutic dose should be about 20-25 mg daily. Since the dose of methandrostenolone cannot be calculated on the basis of sensitivity to it of the tumor tissue because it has never been used as an antitumoral drug, we made our calculations from the data in the literature (See "Pharmaceuticals", M.D. Mashkovsky, "Medizina" Publishers, M., 1985, part I, p. 317) and direct clinical observations in treating volunteers. It was thus established that in neglected cases with complete loss of hormone susceptibility by the tumors it was necessary to use methandrostenolone in a daily dose of 5-10 mg in combination with phthivazid enhancing the conductive effect of this hormone. For phthivazid the daily dose was calculated from the experimental one with due consideration of 10-fold lower metabolism intensity in humans as compared with small mammals. If a dose for an experimental animal (rat) was 45-50 mg/kg body weight, for man it would be 4.5-5 mg/kg body weight, or for a person weighing 60-80 kg or more it would be about 300-500 mg (0.3-0.5 g) per day.

The studies of the antiblastic properties of the new pharmaceutical in the clinic, mainly in incurable volunteer patients yielded the following data significantly distinguishing it from the prototype.

1. The direct antitumoral effect of the proposed pharmaceutical in the form of a mechanical mixture of DL-valine and a low nontherapeutical dose of methyltestosterone is attested by the results of cytological examinations of aspiration specimens from the cavity of the uterus after administration of this pharmaceutical in cases of adenocarcinoma of the uterus body in female volunteers given no specific treatment in the pre-operation period with the exception of DL-valine in combination with a low dose of methyltestosterone. In all 9 patients this treatment resulted in a significant reduction of the tumor, sizes as well as cessation of pains and bloody secretions. In aspiration specimens collected from several of these patients at different intervals (10 days, 2 weeks, 4 weeks) we found dystrophically altered adenocarcinoma cells with significant valuolation of the cytoplasm and nuclei, "corroded" outlines of nuclei, lysis of some nuclei and marked vacuole dystrophy (see Fig. 2).

2. In neglected cases recognised as completely incurable, administration of the new preparation as a mixture of DL-valine with methyltestosterone or with methandrostenolone and phthivazid resulted in a significant prolongation of the survival time of the patient with satisfactory subjective condition and in some cases with the objective antiblastic effect - resolution of the tumors.

3. The action of the new antitumoral pharmaceutical as early as within the first 7-10 days completely eliminated the pain syndrome even in those cases where strong narcotics helped only within a limited time, for instance in neglected cases with metastases into bones, etc.

4. The action of the new pharmaceutical in the course of treatment is not accompanied by any signs of toxicity (hemodynamic disorders, nausea, vomiting).

5. In the course of treatment with the new pharmaceutical, the antitumor resistance increased even in incurable patients as manifested by the improvement in the peripheral blood composition, weight gain, improved appetite, increased working capacity.

6. A significant advantage of this new pharmaceutical consists in the possibility of its successful use in outpatient wards which considerably distinguishes the new pharmaceutical from the majority of the known chemotherapeutical drugs exerting severe side-effects and thereby requiring hospital conditions for their application.

Side Effects

Because of the harmlessness of the new pharmaceutical, there are no significant complications. A long-term (up to 2 months) administration of methyltestosterone in low doses may cause weak manifestations of virilism which rapidly disappear after methyltestosterone administration is discontinued.

If the new pharmaceutical is administered in excessive amounts and the protein diet is not observed, there may occur insignificant nausea, loss of appetite, loss of weight. These signs of amino acid disbalance disappear after reduction of the daily dose of DL-valine and strong observance of the protein diet.

Contraindications

1. Preceeding androgen therapy.
In such cases a low dose of methyltestosterone of the 1st therapy schedule cannot realize its effect of DL-valine conductor in the tumor tissue cells, and there is no antiblastic effect. The 2nd therapy schedule (with methandrostenolone and phthivazid) may be used.

2. The presence of metastases in the lungs, because rapid regression of tumors may result in defects in the walls of the large blood vessels producing life-threatening bleedings.

3. Various forms of stress effect on the patient and mechanical damages preceeding the treatment: recent operational intervention, burns, fractures, etc., which is associated with the property of predominant transportation of DL-valine amino acid to the damaged tissues of the body and not to the tumor tissue.
Therefore the application of the new pharmaceutical is possible only after complete healing of the tissues (20-25 days after operation) and cessation of the stress condition. Blood transfusion (especially multiple) also exerts an effect close to that of the stress on the metabolic processes. Therefore the administration of the pharmaceutical must be discontinued for this time.

4. During the treatment, the use of diuretics affecting the salt metabolism must be avoided.

5. No other antitumoral drugs should be given during the treatment with the new pharmaceutical.

6. During the treatment with the new pharmaceutical the administreation of other hormones, adaptogenes, phytotherapy, drugs stimulating homopoiesis, cardiac activity must be discontinued.

Examples

The invention is illustrated by the following specific examples of the antitumoral properties of the alleged new pharmaceutical.

1. Patient Melnikova, G.A., 75 years old, case history No. 12763/a. Exploratory laparotomy was done on 31.05.1977 at the gynecology department of Rostov Research Institute of oncology. A metastasis from the greater omentum was taken for histological analysis No. 104847-848. Clandular solid ovary cancer.

Clinical diagnosis: ovary cancer of IV stage with invasive tumor growth and outgrowth into the intestines, with metastases into the omentum and in the liver, with ascitic fluid accumulation in the abdominal cavity.

In the post-operation period, a course of chemotherapy (im., thio-tepa) was given as a result of which no ascites accumulated but the tumor growth continued. In July, the patient was discharged from the hospital and prescribed symptomatic treatment.

In late July, 1977, as a volunteer, the patient, weighing 54 kg, began the treatment with the proposed pharmaceutical in the form of a mixture of 2 g DL-valine in combination with 25 mg (0.025 g) or 5 tablets methyltestosterone per day, with strict observance of the diet and with the division of the daily dose into 4 single ones taken before beals; 0.5 g DL-valine in warm milk enterally and 1 tablet of methyltestosterone sublingually 4 times, and 1 tablet of methyltestosterone sublingually in the middle of the day without DL-valine. The duration of the course - 2 months.

During this time the pains in the low abdomen and in the area of the liver ceased completely, the gastrointestinal tract function normalized (constipations and bloody secretions disappeared), the appetite improved, weight gain was 4.5 kg, the strength and working capacity were restored. No tumor was palpated.

Owing to this status the patient was hospitalized one month later for the 2nd course of chemotherapy (im, thio-tepa, a total dose of 200 mg). Gynecological examinations revealed no neoplasia.

In March and July, 1978, prophylactically 2 more courses of thio-tepa were given which produced stable leukopenia.

In August, 1978 a second course of the proposed pharmaceutical was given by the same schedule for 1 month. The blood analysis was normal. On the 7th day of the treatment the patient had severe pains in the liver area which persisted for 24 hours but then disappeared completely. Gynecological examination revealed no neoplasia. The patient felt well. No treatment was given until April next year: general condition was satisfactory, gained some weight, gynecological examinations revealed nothing.

In late April-May, 1979, she was given a 5th (prophylactic) course of thio-tepa, im., tolerated satisfactorily.

In June, 1979, she was given a 3rd (prophylactic) course of the new pharmaceutical but in another daily dose because of a significant change of weight in 2 years (from 54 kg to 68 kg), i.e. she gained 14 kg. She was given a mixture of 3 g DL-valine and 25 g methyltestosterone daily in 4 single doses, with the same diet. The course lasted 2 months. There appeared insignificant signs of virility which oppressed the patient because of libido manifestations. Because of this, she declined further prophylactic administration of the new pharmaceutical. Late in 1979, in spring and autumn of 1980 she was given 3 more prophylactic courses of chemotherapy with cyclophosphane which she tolerated with more difficulty.

In February and March 1981 the patient suffered two insults. She died with the second, without any signs of neoplasia which had been established at a gynecological examination one month before death. The relatives refused the autopsy.

The conclusion: an increase in the period of active life by 4 years after recognition of her condition as completely incurable cannot be regarded to be due to the success of traditional cancer chemotherapy alone. The new pharmaceutical not only corrected the side effects of chemical drugs but clearly facilitated the antiblastic effect.

2. Patient Tkachenko, M.A., 28 years old, case history No. 11518/x, admitted to gynecological department of Rostov Research Institute of oncology 15.08.89. The status at admission: within 2 months great enlargement of the abdomen, great weight loss, evening temperature about 39°C, suffocation, X-ray of the chest revealed a metastasis into the pulmonary pleura. Endolymphatic polychemotherapy was started but did not alleviate the condition of the patient.

On 31.08.89 an exploratory laparotomy was performed with evacuation of 15 l of ascitic fluid from the abdodominal, cavity and the about 2 litres of fluid from the pleural cavity.

The diagnosis : ovary cancer of IV stage with out-growth of the tumors into the intestines and with metastases into the greater omentum, liver, peritoneum; pleura (metastatic pleuritis). Histological analysis No. 261362: glandular-capillary cancer.

On 13.09.89 a preparation of platinum was inoculated intraperitoneally. After this the condition of the patient deteriorated even more, she was considered to be incurable and was discharged from the hospital in the pre-terminal condition.

On 26.09.89 she began, as a volunteer, the treatment with the new pharmaceutical in the outpatient department. The daily dose (with the patient's weight about 70 kg) consisted of 3 g DL-valine in combination with 25 mg (0.025 g or 5 tablets) methyltestosterone. The daile dose was divided into 3 single ones (1 g DL-valine in warm milk enterally and 1 tablet of methyltestosterone sublingually 3 times daily - on an empty stomach and before meals; and 1 tablet of methyltestosterone without DL-valine sublingually 2 times in the middle of the day before meals). The patient strictly observed the diet with the excess of protein, limited amount of carbohydrates and complete exclusion of animal fat and phospholipids (fish hard-roe, egg yolk), using no more than 1-2 table spoonfuls of vegetable oil daily.

Within the first 10-15 days of treatment with the net, pharmaceutical the following changes occured in the condition of the patient:

1. Vomiting stopped and intestinal evacuation occurred independently;

2. The body temperature became normal;

3. The urine colour became lighter (previously - dark beer colour);

4. The skin colour changed from dray-green into yellowish-white.

5. The liver enlarged slightly and periodically was painful;

6. Tachycardia appeared from time to time;

7. The patient showed good appetite and interest for life.

The course of treatment was 1 month because the patient was considered to be curable and was urgently hospitalized on 23.10.89 for a repeated intraperitoneal injection of platinum preparation. The patient bore the injection of the platinum preparation easier than the first time, but thereagain appeared sings of cardio-pulmonary insufficiency.

After the discharge from the hospital, on 09.11.89 the patient again began to get the treatment with the new pharmaceutical, a 2-month course (November-December, 1989). Changes in this period:

1. Signs of cardio-pulmonary insufficiency disappeared.

2. Periodically occuring pains in the liver stopped but the liver was still enlarged.

3. No signs of ascites either in the abdominal or in pleural cavity.

4. The appetite is still good, gain in weight - 4 kg.

5. The patient takes active part in life, wants to go to work but she gets tired quickly.

In January 1990, she was hospitalized for 3 week-course of systemic chemotherapy (im. thio-tepa) which was tolerated satisfactorily, without disorders in hemodynamics, without nausea and vomiting. But the course was not completed and discontinued because of a severe allergic edema of Quincke's type. At this time pains in the right and left hypochondrium.

Early in February, 1990 a two-month course (February-March) of treatment with the new pharmaceutical was started in the same doses as before. After the course:

1. The objective status: as shown by gynecological examination, in the small pelvis all tumoral formations with the exception of a small tumor on the right side (of the size of a child's fist) resolved; no signs of metastatic pleuritis.

2. The subjective status is good. Plans to get married. Early in April she was married, refused any treatment for April and May. Menses absent for 8 months started on May 1.

In the mid-may, 1990, however, there appeared signs of metastatic pleuritis: hard breathing, fluid in the left lung up to the 3rd rib. No changes in the small pelvis.

From May 18, 1990, she was given a course of systemic chemotherapy with cyclophosphane in an outpatient ward with no result: pleuritis progressed (fluid up to the 5th rib).

On June 4, 1990, the 4th course of therapy with the new pharmaceutical was started with a daily dose of DL-valine 3 g and methyltestosterone 3 tablets (0.015 g), because the patient, due to a very high libido, refused to take 5 tablets per day (0.025 g), intending otherwise to discontinue any therapy. In 2 weeks of the treatment pleuritis decreases (again to the 3rd rib), respiration became easier.

By the request of the physician-in-charge, chemotherapy with cyclophosphane (2 weeks) was alternated with the treatment using the new pharmaceutical (2 weeks). After two such alternations the patient refused to take more chemotherapy with cyclophosphane because each time it caused very strong allergic edema of pudendal lips and larynx not alleviated by the new pharmaceutical.

For a year (August, 1990 - August, 1991) the patient carried out independent treatment with the new pharmaceutical in courses of 1-2 months in the same doses with approximately one-month intervals. However, the generalization of the process continued slowly: there appeared a metastasis in the left breast, in the interpleural area there accumulated the fluid which in this period was removed 3 times.

Since it appeared definite that malignant neoplasias by the end of that year filling the small pelvis had completely lost susceptibility to hormones,on August 13, 1991, the treatment with the mechanical mixture was started: 3 g of DL-valine, 0.005 g methandrostenolone, and 0.3 g phthivazid per day. All the mixture was divided into 2 single doses in the morning and the evening; each consisting of 1.5 g DL-valine and 1/2 tablet methandrostenolone, 1/2 tablet phthivazid with warm milk on an empty stomach, a course of 1 month.

This treatment apparently caused extensive necrotic generation of metastases in the interpleural cavity: the temperature rose high by the evening, severe coughing developed, hard breathing. Vomiting occurred once in 1-2 days, the vomitus containing dark structureless masses; after this the fever dropped, breathing became easier, but coughing did not stop.

By the patient's request is September 1991 she was again given a 1-month course of treatment with DL-valine (3 g) in combination with 0.015 g methyltestosterone. Coughing slightly decreased, vomiting stopped, however, the generalization of the process continued: the metastasis in the breast began to grow out into the back muscles.

Early in October another course was started with a mixture of DL-valine, methandrostenolone and phthivazid in the sale dose. The coughing increased, frequent episodes of suffocation developed,attempts at the fluid pumping out showed the absence of the latter: apparently the cavity was filled with tumor masses. Cardiac weakness increased. No pains. The patient seldom left the house because of difficulties in climbing the stairs, but at home she read, wrote, freely moved about the room.

On November 8, 1991 the patient died of a heart attack for 30-40 min. She had lived more than 2 years from the time she had been considered to be incurable.

The conclusion:

1. The success in prolongation of the active life of this young woman cannot be associated with the treatment with the new pharmaceutical alone because in the 1st year she received the conventional chemotherapy courses which she could hardly tolerate each time.

2. The turning point towards the new generalization of the process fell on April-May when all the treatment was discontinued. This should not have been done. Since our treatment was only voluntary we had no right to insist.

3. The use of the mixture of DL-valine, methandrostenolone and phthivazid, in our opinion, was too delayed and the time had probably been lost for its optimal effect.

The new pharmaceutical with the antitumoral activity in the for of a mixture of DL-valine and small doses of methyltestosterone was initially tried in 33 patients (volunteers) with malignant tumors of genitalia confirmed histologically as cancer of the following localizations: malignant ovary cancers - 9 patients, malignant tumors of the uterus body - 21 patients, cancer of the uterine tube - one patient, cervical cancer - 2 patients.

With the exeption of 9 patients with the uterus body cancer in the II-III stage of the disease for whom the new pharmaceutical was used as the first stage of treatment in the pre-operation period, all the other patients were in the III-IV stage of the disease and by the time of treatment with the new pharmaceutic they had been given the conventional treatment or chemotherapy alone, without any effect, having large immovable tumor conglomerates in the small pelvis oe exudates into serious cavities. Some of them had relapses of the disease after ineffective combined treatment.

Most of the patients treated with the new pharmaceutical a good (tumors resolved) or satisfactory effect. Complete lack of effect was observed is 5 patients only. In four of them the tumors were definitely hormone-unsusceptible: cervical cancer, uterine tube cancer, sarcoma of the uterus body, and the fifth patient was presented for treatment in a very severe condition due to rapid generalization of the process.

The proposed pharmaceutical was also tested in 5 incurable patients suffering from the mammary gland cancer with metastases into bones, with growing out metastases into the muscles of skin of the chest who were discharged for symptomatic treatment in a very severe condition after ineffective irradiation therapy and 10-12 courses of chemotherapy.

In one of these cases, after a long-term hospital treatment metastases into the bones of the skull, pelvis, ribs led to the pre-terminal condition of the patient and she was brought from the hospital on the stretchers. The effect of the treatment with the mechanical mixture of 2 g DL-valine and 20 mg methyltestosterone was both subjective and objective. Severest pains usually accompanying such metastasising which coild only be stopped for $1\frac{1}{2}$-2 hours by trimeperidine hydrochloride (promedol), began to subside since the 3rd day of treatment and completely disappeared within 2 weeks. As a result, in a month the patient started to get up from the bed and grudually became involved in the active life. Every 2 months, the affected parts of the skeleton were examined by roentgen. Signs of beginning reparation of the bone tissue were observed in skull and pelvic bones. With short intervals of $1$-$1\frac{1}{2}$ weeks the patient took the treatment in the outpatient ward for 8 months, until she died of thromboembolism as a result of long-term uncontrolled taking of calcium chloride for 3 months.

Until the last day this patient actively did the house work, went shopping and to the market!

In 3 cases, treatment with the mechanical mixture of DL-valine, methandrostenolone and phthivazid resulted in temporary but well marked subjective improvement in the condition of the patients which finally ended after another course of chemotherapy because they were again hospotalized into chemo-hormone therapy department of Rostov Research Institute of Oncology.

In one case, such subjective considerable improvement stopped sharply during the treatment with the proposed pharmaceutical because of the stress condition of the patient developing due to her personal problems. She was not hospitalized.

The observations carried out by the author showed the treatment with the proposed pharmaceutical to give mainly satisfactory results even after previously performed ineffective chemo-radiation therapy.

The technical result of the invention consists in the following.

The pharmacological properties of tile proposed pharmaceutical studied experimentally in induced hormone-susceptible tumors of mammary cancer in rats and in normal animals, as well as the data obtained clinically in treatment of volunteers in incurable cases attest to its complete harmlessness, sufficiently marked antiblastic properties with simultaneous stimulation of the defence forces of the body and analgetic effect.

Legends for Figures

Fig. 1     - DMBA-induced mammary gland cancer in rats

1. Control - glandular-solid cancer of the mammary gland in rat (untreated). Magnification x 160.

2. Experiment - after treatment with DL-valine in mixture with methyltestosterone (regression). Marked dystrophic lesions in tumor cells: predominant vacuolation of the cytoplasm and lysis of both nucleus and cytoplasm. Magnification x 200.

Fig. 2     - Adenocarcinoma of human uterine body. Aspiration from the uterine cavity (cytology)

1. Before treatment. Rosette structures from elongated a typical cells of the cylinder shape (magnification x 600, im.).

2. Treatment for 2 weeks with DL-valine and methyltestosterone. Vacuolation of the cytoplasm and

nuclei of tumor cells (magnification x600, im). 3. Treatment for 4 weeks with DL-valine and methyltestosterone. Marked dystrohy of vacuoles: fusion of small vacuoles in cytoplasm and nuclei of tumor cells (magnification x 600, im.).

4. Treatment for 10 days. A layer of dystrophically changed cancer cells: "corroded" outlines of the nuclei, lysis of individual nuclei (magnification x 600, im.).

## Claims

1. What is claimed is a pharmaceutical exerting antitumoral activity which consists of a mechanical mixture of 2-4 g DL-valine and a low nontherapeutical dose of 0.020-0.025 g methyltestosterone for treatment of hormone-susceptible cancer tumors in women, and a mechanical mixture of 2-4 g DL-valine, 0.005-0.010 g methandrostenolone and 0.3-0.5 g phthivazid for treatment of cancer tumors without hormone-susceptibility.

# INTERNATIONAL SEARCH REPORT

International application No.

PCT/RU 93/00139

**A. CLASSIFICATION OF SUBJECT MATTER**

Int.Cl.$^5$: A 61 K 31/195, 31/565, 31/44

According to International Patent Classification (IPC) or to both national classification and IPC

**B. FIELDS SEARCHED**

Minimum documentation searched (classification system followed by classification symbols)

Int.Cl.$^5$: A 61 K 31/195, 31/44, 31/565

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | EP, B1, 0280593 (ROUGEREAU, ANDRE), 31 August 1988, the claims | 1 |
| A | FR, A1, 2609893 (ROUGEREAU, ANDRE), 29 July 1988, the claims | 1 |
| A | M. D. MASHKOVSKY: "Lekarstvennye sredstva", 1986, Meditsina (Moscow), page 603 | 1 |

☐ Further documents are listed in the continuation of Box C.  ☐ See patent family annex.

| Date of the actual completion of the international search | Date of mailing of the international search report |
|---|---|
| 21 September 1993 (21.09.93) | 5 November 1993 (05.11.93) |

| Name and mailing address of the ISA/ <br><br> **RU** | Authorized officer |
|---|---|
| Facsimile No. | Telephone No. |

Form PCT/ISA/210 (second sheet) (July 1992)